# NOUVEAU FASCICULE DE BREVET EUROPEEN

(11) **EP 1 053 745 B2**
(45) Date de publication et mention de la décision concernant l'opposition: **28.04.2010**
(45) Mention de la délivrance du brevet: 09.08.2006
(21) Numéro de dépôt: 00401167.2
(22) Date de dépôt: 27.04.2000
(51) Int. Cl.: A61Q 19/08, A61K 8/41

(54) **Utilisation d'au moins un inhibiteur d'au moins un canal calcique dans le traitement des rides**
Anwendung von mindestens einem Calcium-Kanal-Hemmstoff zur Behandlung von Hautfalten
Use of at least a calcium-channel-inhibitor in the treatment of wrinkles

(30) Priorité: 18.05.1999 FR 9906290
(43) Date de publication de la demande: 22.11.2000
(73) Titulaire: L'Oréal, 75008 Paris (FR)
(72) Inventeur: Breton, Lionel, 78000 Versailles (FR); Nonotte, Isabelle, 75017 Paris (FR)
(74) Mandataire: Le Coupanec, Pascale A.M.P.

(56) Documents cités:
- EP-A1- 0 439 640
- EP-A1- 0 797 985
- EP-A2- 0 508 324
- WO-A-91/02497
- WO-A-97/18782
- WO-A1-94/26308
- WO-A1-96/19184
- BE-A- 1 003 002
- DE-A1- 3 940 315
- JP-A- 01 238 509
- US-A- 5 059 606
- US-A- 5 214 041
- US-A- 5 554 608
- PATENT ABSTRACTS OF JAPAN vol. 012, no. 230 (C-508), 29 juin 1988 (1988-06-29) & JP 63 022007 A (KANEBO LTD), 29 janvier 1988 (1988-01-29)
- PATENT ABSTRACTS OF JAPAN vol. 012, no. 159 (C-495), 14 mai 1988 (1988-05-14) & JP 62 270519 A (KANEBO LTD), 24 novembre 1987 (1987-11-24)
- PATENT ABSTRACTS OF JAPAN vol. 017, no. 067 (C-1025), 10 février 1993 (1993-02-10) & JP 04 275217 A (NIPPON MINING CO LTD), 30 septembre 1992 (1992-09-30)
- 'Römpp Chemie Lexikon', vol. 2, 1995, GEORG THIEME VERLAG, STUTTGART article 'Glykolsäure', pages 1618 - 1618
- 'J. Physiol.', vol. 260, 1976 article S.G.CULL-CANDY ET AL: 'Effects of Botulinum Toxin on Neuromuscular Transmission in the Rat', pages 177 - 203
- 'Molekularbiologie der Zelle, 2.Auflage', 1990, VCH, WEINHEIM article B. ALBERTS ET AL, pages 375 - 380
- A. BLITZER ET AL: 'Botulinum Toxin for the Treatment of Hyperfunctional Lines of the Face' ARCH OTOLARYNGOL HEAD NECK SURG. vol. 199, Septembre 1993, pages 1018 - 1022
- L. C. SELLIN: 'The Action of Botulinum Toxin at the Neuromuscular Junction' MEDICAL BIOLOGY vol. 59, 1981, pages 11 - 20
- B. POULAIN ET AL: 'Heterologous Combinations of Heavy and Light Chains from Botulinum Neurotoxin A and Tetanus Toxin Inhibit Neurotransmitter Release in Aplysia' THE JOURNAL OF BIOLOGICAL CHEMISTRY vol. 266, no. 15, 1991, pages 9580 - 9585
- 'Lexikon der Hilfsstoffe für Pharmazie, Kosmetik und angrenzende Gebiete', vol. 9, 1971, EDITION CANTOR AG, AULENDORF article H. P. FIEDLER: 'Chelatbildner, Chelating agents, Chelation', pages 118 - 119
- 22 Avril 2007, WIKIPEDIA WEB-SEITE article 'Kalziumantagonist'
- 'Römpp Chemie Lexikon CD-ROM', Juillet 2006, GEORG THIEME VERLAG article 'Calciumkanal-Blocker'
- L.L. SIMPSON:: 'Botulinum Neurotoxin and Tetanus Toxin', 1989, ACADEMIC PRESS article L.L. SIMPSON ET AL: 'Peripheral Actions of the Botulinum Toxins', pages 153 - 178

## Description

La présente invention se rapporte à l'utilisation non thérapeutique dans une composition cosmétique d'une quantité efficace d'au moins un inhibiteur d'au moins un canal calcique telle que définie en revendication 1.

Les femmes, voire même les hommes, ont tendance actuellement à vouloir paraître jeunes le plus longtemps possible et cherchent par conséquent à estomper les marques du vieillissement de la peau, qui se traduisent notamment par des rides et des ridules. A ce sujet, la publicité et la mode font état de produits destinés à garder le plus longtemps possible une peau éclatante et sans ride, marques d'une peau jeune, d'autant plus que l'aspect physique agit sur le psychisme et/ou sur le moral.

Jusqu'à présent, on traitait les rides et les ridules à l'aide de produits cosmétiques contenant des actifs agissant sur la peau, par exemple en l'hydratant ou en améliorant son renouvellement cellulaire ou encore en favorisant la synthèse du collagène qui compose le tissu cutané. Mais, à ce jour on ne sait pas agir sur les rides en intervenant sur les éléments contractiles présents dans la peau.

Ainsi, il est connu que les muscles peauciers du visage sont sous le contrôle de afférences nerveuses motrices du nerf facial et que, par ailleurs, les cloisons interlobulaires de l'hypoderme contiennent en leur sein des fibres qui constituent un tissu musculaire strié (panniculus carnosus). D'autre part, il est également connu qu'une sous-population de fibroblastes du derme, que l'on appelle myofibroblastes, présente des caractéristiques contractiles communes avec le tissu musculaire.

Le calcium est le messager final de la contraction musculaire. Le cycle contraction-relâchement est dû aux variations de la concentration du calcium cytoplasmique de 10⁻⁸ à 10⁻⁵ M dans la cellule contractile.

Dans le muscle au repos, la concentration intracellulaire du calcium libre reste inférieure à 10⁻⁸ M bien que la concentration extracellulaire soit 10.000 fois plus élevée et que la force représentée par le gradient de potentiel électrochimique tende à faire pénétrer le calcium dans la cellule. Cet état de repos est dû à la faible perméabilité au calcium de la membrane cellulaire et à l'activité de divers mécanismes qui séquestrent le calcium ou l'expulsent de la cellule. Diverses protéines cytoplasmiques, notamment les parvalbumines ont ainsi la capacité de lier le calcium. Parmi les organelles intracellulaires, le réticulum endoplasmique peut accumuler et relarguer le calcium dans des conditions compatibles avec une régulation physiologique.

L'élévation du taux de calcium dans le cytoplasme du myocyte permet l'activation de la machinerie contractile. L'entrée du calcium dans le compartiment intracellulaire (dépolarisation) participe à la diminution de la différence de potentiel entre l'extérieur et l'intérieur et rend ainsi la cellule plus excitable.

En effet, la dépolarisation des tubules transverse (invagination de la membrane cellulaire) qui se propage aux tubules longitudinaux (reticulum sarcoplasmique) induit la libération momentanée du calcium intracellulaire par ces dernières. En présence de calcium les protéines contractiles du muscle strié présentent une activité ATPase qui fournit l'énergie nécessaire à la contraction.

A l'inverse, le relâchement du muscle strié a lieu quand une nouvelle molécule d'ATP se fixe aux protéines contractiles. Le calcium intracellulaire réintègre alors le compartiment intracellulaire et sa concentration redevient proche d'une valeur de 10⁻⁸M.

Par ailleurs, il a été montré que la toxine botulique, utilisée à l'origine pour traiter les spasmes, pouvait agir sur les états de spasticité musculaire (voir A. Blitzer et al., Arch. Otolaryngol. Head Neck Surg., 1993, 119, pages 1018 à 1022) et sur les rides de la glabelle qui sont les rides inter sourcilières (voir J.D. Carruters et al., J. Dermatol. Surg. Oncol., 1992, 18, pages 17 à 21). En conséquence, il est possible d'agir sur la composante nerveuse des rides.

Dans le système nerveux périphérique, la jonction entre un nerf et un muscle constitue la plaque neuro-musculaire, en amont de laquelle se trouve la voie nerveuse efférente appelée motoneurone. Par ailleurs, les membranes cellulaires de chaque fibre nerveuse comportent également de nombreux canaux ioniques, et notamment des canaux calciques, aptes à laisser traverser l'élément correspondant sous forme ionique, dans ce cas particulier le calcium.

On comprend ainsi le rôle important du calcium et de la régulation de sa concentration intracellulaire dans les phénomènes de contraction/relâchement musculaires.

La régulation de la concentration intracellulaire du calcium n'est possible que parce que l'efflux de calcium corrige l'influx. Ceci ne peut être assuré que par une expulsion du calcium cellulaire par un ou des mécanismes aptes à surmonter le gradient de potentiel électrochimique évoqué plus haut.
Deux types de mécanismes peuvent intervenir : une pompe à calcium qui expulse activement les cations aux dépens de l'hydrolyse d'ATP et un mouvement de calcium couplé à un mouvement de sodium. Dans la plupart des cellules, la pompe à calcium ATP-dépendante opère plus efficacement en présence de la calmoduline qui augmente son affinité.

Afin de mieux décrire les changements de perméabilité au calcium, il est actuellement habituel de considérer que cette perméabilité correspond à l'ouverture de canaux calcium membranaires, canaux opérés par des variations du potentiel de la membrane (VOC) ou l'activation des récepteurs membranaires (ROC). A ce jour, six types VOC de canaux calcium (L, N, T, P, Q, R) ont été identifiés.

On comprend donc de ce qui précède que la contraction ou l'hypercontraction de certains muscles de la face résulte en l'apparition de rides. Cette activation musculaire est elle-même induite par une variation de flux de calcium au travers des canaux calciques transmembranaires.

Or la demanderesse a maintenant découvert après de nombreux tests cliniques, que la fibre musculaire contractile, qui se trouve sous le contrôle direct de l'influx neuro-moteur, joue un rôle essentiel dans la formation des rides et que la modulation de l'influx neuro-moteur et le contrôle de la contraction des fibres musculaires, jouent un rôle essentiel dans la formation des rides. Ainsi la modulation de la contraction motrice atténue non seulement les rides mais également les ridules et présente aussi un effet de « lissage » sur le microrelief cutané. Elle a aussi trouvé que les tissus cutané et sous-cutané comportent des canaux calcium, ce qui, jusqu'à présent, n'a pas été envisagé.

Personne n'a, jusqu'à ce jour, établi un lien entre les canaux calcium du tissu nerveux et/ou musculaire sous cutanés et les rides, et n'a trouvé qu'il est possible de traiter les rides en agissant sur les canaux calcium.

Ainsi, la demanderesse propose pour relâcher ou relaxer les tissus, et ainsi diminuer les rides et les ridules, d'agir sur les canaux calciques.

Dès 1965, des travaux ont été menés par T. Godfraind afin de rechercher les mécanismes par lesquels certaines substances médicamenteuses inhibaient la réponse contractile à plusieurs agents vasoactifs. L'hypothèse proposée était que la perméabilité de la membrane au calcium pourrait être inhibée par des agents pharmacologiques, ce qui constituerait le mécanisme commun sur lequel agiraient des antagonistes polyvalents.
La technique expérimentale la plus simple, permettant de montrer qu'un agent pharmacologique est à même d'inhiber l'entrée du calcium, consiste à préincuber un muscle lisse dans une solution physiologique dépourvue de calcium, de la dépolariser dans une solution riche en KCI et d'augmenter graduellement la concentration de calcium dans la solution de perfusion. Ceci provoque une augmentation de tension du muscle dont la valeur évolue jusqu'à un maximum en fonction de la concentration de calcium. Lorsque ce protocole est répété en présence d'une substance supposée inhiber l'entrée de calcium ainsi que cela fut réalisé pour la première fois avec la cinnarizine, les réponses contractiles sont inhibées d'une manière dose-dépendante. Un concept semblable a été appliqué pour décrire l'action du vérapamil sur le coeur. Le vérapamil a été d'abord considéré comme un β-bloquant, son action est plus complexe puisqu'il exerce une action inhibitrice sur le couplage excitation-contraction. Sur le muscle papillaire, le vérapamil abolit la contraction en modifiant très faiblement le potentiel d'action. C'est cette observation qui conduit à considérer le vérapamil comme un antagoniste du calcium.

Ainsi, la présente invention se rapporte à l'utilisation non thérapeutique dans une composition cosmétique d'une quantité efficace d'au moins un inhibiteur d'au moins un canal calcique, l'inhibiteur étant destiné à prévenir et/ou traiter les rides et les ridules de la peau par relaxation et/ou relâchement du tissu cutané et/ou sous cutané et étant choisi parmi le vérapamil, l'anipamil, le gallopamil, le dévapamil, le falipamil, le tiapamil, la nifédipine, l'amlodipine, la dazodipine, la félodipine, l'isradipine, lanicardipine, la nimodipine, la nisoldipine, la nitrendipine, la ryosidine, le Diltiazem, et la cinnarizine.

Cette utilisation s'avère particulièrement efficace pour diminuer les rides et ridules.

Plus particulièrement la relaxation et/ou le relâchement du tissu cutané et/ou sous-cutané correspond à un relâchement ou une relaxation musculaire.

Pour qu'une substance soit reconnue comme un inhibiteur des canaux calcium, autrement appelé dans le texte inhibiteur calcique, elle doit pouvoir diminuer la concentration intracellulaire en calcium ou diminuer la liaison du calcium aux protéines intracellulaires comme par exemple la calmoduline, tel que cela est notamment décrit par exemple, par Galizzi, J.P et *al*, J. Biol. Chem. 1987, 262 p 6947 ou Y. Okamiya et *al*, Eur. J. Pharmacol. 1991, 205, p 49 ou J.A. Wagner et al, J. Neurosci. 1988, 8, p 3354 ou H.R Lee et *al*, Life Sci. 1984, 35 p 721 ou Schoemaker H. et Lauger S. Eur. J. Pharmacol. 1985, 111 p 273 ou encore I.J. Reynolds et *al*, J. Pharmacol. Exp. Ther. 1986, 237 p 731.

Une substance est reconnue comme relaxante au sens de l'invention lorsqu'elle montre un effet de relaxation sur un tissu musculaire contracté et/ou montrer un effet inhibiteur dans un modèle expérimental de jonction nerf-muscle (plaque motrice) notamment dans le modèle décrit par W. Steinbrecher dans : Electrodes for stimulation and bioelectric potentiel recording, Ed. Biomerstechnich, 1988, pages 96-98.

La quantité efficace d'inhibiteur d'au moins un canal calcique utilisable selon l'invention est bien entendu fonction de l'effet recherché et peut donc varier dans une large mesure.

Pour donner un ordre de grandeur, il est possible d'utiliser selon l'invention un inhibiteur d'au moins un canal calcique en une quantité représentant de 0,0001% à 10% du poids total de la composition et préférentiellement en une quantité représentant de 0,001% à 5% du poids total de la composition.

Très préférentiellement selon l'invention on utilise les inhibiteurs de l'entrée du calcium comme le vérapamil.

Les compositions selon l'invention peuvent se présenter sous toutes les formes galéniques normalement utilisées pour une application topique, injectable ou orale.

La composition selon l'invention peut être appliquée soit par voie locale, c'est-à-dire par voie topique, ou par injection sous-cutanée et/ou intradermique.

Préférentiellement selon l'invention, la composition est appliquée par voie topique.

Les quantités des différents constituants des compositions selon l'invention sont celles classiquement utilisées dans les domaines considérés et sont appropriées à leur forme galénique.
Pour une application topique, les compositions de l'invention comprennent un milieu compatible avec la peau. Ces compositions peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Ces compositions à application topique peuvent constituer notamment une composition de protection, de soin pour le visage, pour le cou, pour les mains ou pour le corps, (par exemple crèmes de jour, crèmes de nuit, crèmes ou huiles solaires, laits corporels), une composition de maquillage (par exemple fond de teint) ou une composition de bronzage artificiel.

Quand la composition de l'invention est une émulsion, la proportion de corps gras qu'elle contient peut aller de 5 % à 80 % en poids, et de préférence de 5 % à 50 % en poids par rapport au poids total de la composition. Les corps gras et les émulsionnants utilisés dans la composition sous forme d'émulsion sont choisis parmi ceux classiquement utilisés dans le domaine cosmétique ou pharmaceutique.

Comme corps gras utilisables dans l'invention, on peut citer les huiles minérales (vaseline), les huiles végétales (fraction liquide de beurre de karité) et leurs dérivés hydrogénés, les huiles animales, les huiles de synthèse (perhydrosqualène), les huiles siliconées (diméthylpolysiloxane) et les huiles fluorées. Comme autres corps gras, on peut encore citer les alcools gras (alcool cétylique, alcool stéarylique), les acides gras (acide stéarique) et les cires.

Les émulsionnants peuvent être présents, dans la composition, en une proportion allant de 0,3 % à 30 % en poids, et de préférence de 0,5 à 30 % en poids par rapport au poids total de la composition.

De façon connue, la composition de l'invention peut contenir également des adjuvants habituels dans les domaines correspondants, tels que les gélifiants hydrophiles ou lipophiles, les conservateurs, les antioxydants, les solvants, les parfums, les charges, les filtres et les matières colorantes. Par ailleurs, ces compositions peuvent contenir des actifs hydrophiles ou lipophiles. Les quantités de ces différents adjuvants ou actifs sont celles classiquement utilisées dans le domaine cosmétique ou pharmaceutique, et par exemple de 0,01 % à 20 % du poids total de la composition. Ces adjuvants ou ces actifs, selon leur nature, peuvent être introduits dans la phase grasse, dans la phase aqueuse et/ou dans des vésicules lipidiques.

Parmi les actifs que peuvent contenir les compositions de l'invention, on peut notamment citer les actifs ayant un effet sur le traitement des rides ou des ridules, et en particulier les actifs kératolytiques. Par kératolytique, on entend un actif ayant des propriétés desquamantes, exfoliantes ou gommantes, ou un actif capable de ramollir la couche cornée.

Parmi les actifs ayant un effet sur le traitement des rides ou des ridules que peuvent contenir les compositions de l'invention, on peut en particulier citer les hydroxyacides et les rétinoïdes.

Les hydroxyacides peuvent être par exemple des α-hydroxy-acides ou des β-hydroxy-acides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Les hydroxyacides qui peuvent être utilisés sont notamment les acides glycolique, lactique, malique, tartrique, citrique, hydroxy-2 alcanoïque, mandélique, salicylique, ainsi que leurs dérivés alkylés comme l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthyl-benzoïque, ou encore leurs dérivés alcoxylés comme l'acide 2-hydroxy-3-méthoxybenzoïque.

Les rétinoïdes peuvent être notamment l'acide rétinoïque (all-trans ou 13-cis) et ses dérivés, le rétinol (vitamine A) et ses esters tels que le palmitate de rétinol, l'acétate de rétinol et le propionate de rétinol, ainsi que leurs sels.

Ces actifs peuvent être utilisés en particulier à des concentrations allant de 0,0001% à 5% en poids par rapport au poids total de la composition.

La composition de l'invention peut être une composition cosmétique ou dermatologique.
Préférentiellement la composition de l'invention est une composition cosmétique

L'invention a aussi pour objet un procédé non thérapeutique de traitement cosmétique des rides et/ou des ridules, consistant à appliquer sur la peau une composition cosmétique comprenant dans un milieu cosmétiquement acceptable, une quantité efficace d'au moins un inhibiteur d'au moins un canal calcique choisi parmi le vérapamil, l'anipamil, le gallopamil, le dévapamil, le falipamil, le tiapamil, la nifédipine, l'amlodipine, la dazodipine, la félodipine, l'isradipine, lanicardipine, la nimodipine, la nisoldipine, la nitrendipine, la ryosidine, le Diltiazem, et la cinnarizine.

Par milieu cosmétiquement acceptable, on entend compatible avec la peau, le cuir chevelu et/ou les muqueuses.

Le procédé de traitement cosmétique de l'invention peut être mis en oeuvre notamment en appliquant la composition cosmétique telle que définie ci-dessus, selon la technique d'utilisation habituelle de ces compositions. Par exemple : application de crèmes, de gels, de sérums, de lotions, de laits de démaquillage ou de compositions anti-solaires sur la peau ou de compositions pour spray.

Les exemples et compositions suivants illustrent l'invention sans la limiter aucunement. Dans les compositions les proportions indiquées sont des pourcentages en poids, sauf mention contraire.

### Exemple 1 : mesure de l'activité d'un inhibiteur des canaux calciques dans un modèle de jonction nerf / muscle (jonction neuro-musculaire) obtenu dans une préparation nerf phrénique / diaphragme isolé : recherche d'un effet myorelaxant :

Le nerf phrénique et le diaphragme sont soigneusement isolés et placés dans une cuve de 50 ml remplie de liquide de survie (liquide de Krebs Henseleit) maintenu à une température de 37°C et oxygéné à l'aide d'un mélange d'oxygène 95 % et de CO₂ 5 %.

Les variations de tension du diaphragme sont ensuite enregistrées avec une précharge initiale de plusieurs grammes.

Après une période de relaxation de 30 mn, le diaphragme est stimulé indirectement par l'intermédiaire du nerf phrénique.

Sur chaque préparation, l'effet des produits à tester a été dans un premier temps, évalué sur les contractions induites par stimulation indirecte *via* stimulation sur le nerf phrénique (0.1 à 7 volts, 0.3 ms, 0.1 Hz) aux concentrations croissantes et cumulées de 10⁻⁹ M à 10⁻⁴ M.

En cas d'effet à la fin du test, l'effet des produits à tester a été évalué sur les contractions induites par stimulation directe sur le muscle (5 à 50 volts, 0.3 ms, 0.2 Hz) à la concentration de 10⁻⁴ M uniquement, afin de déterminer le mécanisme d'action du produit testé.

Résultats obtenus dans le modèle de plaque motrice avec le Vérapamil.

| Concentration en vérapamil | % d'inhibition (stimulation indirecte) | % d'inhibition (stimulation directe) |
|---|---|---|
| 10⁻⁴ M | 100 | 22 |

### Exemple 2 : exemples de compositions selon l'invention.

### Composition 1 : Lotion de soin pour le visage

| | |
|---|---|
| Vérapamil | 0,05 % |
| Antioxydant | 0,05 % |
| Conservateur | 0,30 % |
| Ethanol (solvant) | 8,00 % |
| Eau | qsp 100 % |

La lotion obtenue agit sur les rides lors d'une utilisation répétée (application biquotidienne pendant un mois).

### Composition 2 : Gel pour le soin du visage

| | |
|---|---|
| Vérapamil | 0,10 % |
| Hydroxypropylcellulose * | 1,00 % |
| Conservateur | 0,30 % |
| Ethanol (solvant) | 15,00 % |
| Antioxydant | 0,05 % |
| Eau | qsp 100 % |

| | |
|---|---|
| * : Klucel H vendu par la société Hercules (gélifiant). | |

Le gel obtenu agit sur les rides. Il peut être appliqué quotidiennement matin et soir pendant un mois.

### Composition 3 : Crème de soin du visage (émulsion huile-dans-eau)

| | |
|---|---|
| Nimodipine | 1,00% |
| Stéarate de glycérol (émulsionnant) | 2,00 % |
| Polysorbate 60 (Tween 60 vendu par la société ICI) (émulsionnant) | 1,00 % |
| Acide stéarique | 1,40 % |
| Triéthanolamine (neutralisant) | 0,70 % |
| Carbomer (Carbopol 940 vendu par la société Goodrich) | 0,40 % |
| Fraction liquide de beurre de karité | 12,00 % |
| Perhydrosqualène | 12,00 % |
| Conservateur | 0,30 % |
| Parfum | 0,50 % |
| Antioxydant | 0,05 % |
| Eau | qsp 100 % |

On obtient une crème blanche, onctueuse, qui agit sur les rides et les ridules, et que l'on peut appliquer quotidiennement.

### Composition 4 : Crème de soin du visage (émulsion huile-dans-eau)

| | |
|---|---|
| Vérapamil | 0,10% |
| Mono-, distéarate de glycérol | 2,00 % |
| Alcool cétylique | 1,50 % |
| Mélange alcool cétylstéarylique/alcool cétylstéarylique oxyéthyléné 33 OE | 7,00 % |
| Diméthylpolysiloxane | 1,50 % |
| Huile de vaseline | 17,50 % |
| Conservateur | 0,30 % |
| Parfum | 0,50 % |
| Glycérine | 12,50 % |
| Eau | qsp 100 % |

## Revendications

1. Utilisation non thérapeutique dans une composition cosmétique d'une quantité efficace d'au moins un inhibiteur d'au moins un canal calcique, l'inhibiteur étant destiné à prévenir et/ou traiter les rides et les ridules de la peau par relaxation et/ou relâchement du tissu cutané et/ou sous-cutané et étant choisi parmi les agents actifs sur la membrane plasmique, complexant le calcium et/ou inhibiteurs de l'entrée du calcium choisis parmi le vérapamil, l'anipamil, le gallopamil, le dévapamil, le fallpamil, le tiapamil, la nifédipine, l'amiodipine, la dazodipine, la félodipine, l'isradipine, lanicardipine, la nimodipine, la nisoidipine, la nitrendipine, la ryosidine, Dlitlazem, et la cinnarizine.

2. Utilisation selon la revendication précédente, pour diminuer les rides et ridules.

3. Utilisation selon l'une quelconque des revendications précédentes, **caractérisée en ce que** l'inhibiteur d'au moins un canal calcique est utilisé en une quantité représentant de 0,0001 % à 10% du poids total de la composition.

4. Utilisation selon l'une quelconque des revendications précédentes, **caractérisée en ce que** l'inhibiteur d'au moins un canal calcique est utilisé en une quantité représentent de 0,001 % à 5% du poids total de la composition.

5. Utilisation selon l'une quelconque des revendications précédentes, **caractérisée en ce que** l'inhibiteur d'au moins un canal calcique est le vérapamil.

6. Procédé non thérapeutique de traitement cosmétique des rides et/ou des ridules, consistant à appliquer par voie topique une composition cosmétique comprenant dans un milieu physiologiquement acceptable une quantité efficace d'au moins un inhibiteur d'au moins un canal calcique choisi parmi les agents actifs sur la membrane plasmique, complexant le calcium et/ou inhibiteurs de l'entrée du calcium choisis parmi les vérapamil, l'anipamil, le gallopamil, le dévapamil, le fallpamil, le tiapamil, la nifédipine, l'amiodipine, la dazodipine, la félodipine, l'isradipine, lanicardipine, la nimodipine, la nisoidipine, la nitrendipine, la ryosidine, Dlitlazem, et la cinnarizine.

## Claims

1. Non-therapeutic use in a cosmetic composition of an effective amount of at least one inhibitor of at least one calcium channel, the inhibitor being intended to prevent and/or treat skin wrinkles and fine lines by relaxing and/or decontracting cutaneous and/or subcutaneous tissue and being chosen from agents that are active on the plasma membrane, calcium-complexing agents and/or calcium-influx inhibitors chosen from verapamil, anipamil, gallopamil, devapamil, falipamil, tiapamil, nifedipine, amlodipine, dazodipine, felodipine, isradipine, lanicardipine, nimodipine, nisoldipine, nitrendipine, ryosidine, Diltiazem, and cinnarizine.

2. Use according to the preceding claim, for reducing wrinkles and fine lines.

3. Use according to either of the preceding claims, **characterized in that** the inhibitor of at least one calcium channel is used in an amount representing from 0.0001% to 10% of the total weight of the composition.

4. Use according to any one of the preceding claims, **characterized in that** the inhibitor of at least one calcium channel is used in an amount representing from 0.001% to 5% of the total weight of the composition.

5. Use according to any one of the preceding claims, **characterized in that** the inhibitor of at least one calcium channel is verapamil.

6. Non-therapeutic process for the cosmetic treatment of wrinkles and/or fine lines, which consists in topically applying a cosmetic composition comprising, in a physiologically acceptable medium, an effective amount of at least one inhibitor of at least one calcium channel chosen from agents that are active on the plasma membrane, calcium-complexing agents and/or calcium-influx inhibitors chosen from verapamil, anipamil, gallopamil, devapamil, falipamil, tiapamil, nifedipine, amlodipine, dazodipine, felodipine, isradipine, lanicardipine, nimodipine, nisoldipine, nitrendipine, ryosidine, Diltiazem, and cinnarizine.

## Patentansprüche

1. Nichttherapeutische Verwendung einer wirksamen Menge mindestens eines Inhibitors mindestens eines Calciumkanals in einer kosmetischen Zusammensetzung, wobei der Inhibitor dazu vorgesehen ist, die Falten und Fältchen der Haut durch Entspannung und/oder Lockerung des Hautgewebes und/oder des Unterhautgewebes zu behandeln und/oder ihnen vorzubeugen, und ausgewählt ist aus Stoffen, die auf die Plasmamembran wirken, Stoffen, die Calcium komplexierten, und/oder Inhibitoren für den Eintritt von Calcium, ausgewählt aus Verapamil, Anipamil, Gallopamil, Devapamil, Falipamil, Tiapamil, Nifedipin, Amiodipin, Dazodipin, Felodipin, Isradipin, Nicardipin, Nimodipin, Nisoldipin, Nitrendipin, Ryosidin, Diltiazem und Cinnarizin.

2. Verwendung nach dem vorhergehenden Anspruch zur Verminderung von Falten und Fältchen.

3. Verwendung nach einem der vorhergehenden Ansprüche, **dadurch gekennzeichnet, dass** der Inhibitor mindestens eines Calciumkanals in einer Menge von 0,001 bis 10 % des Gesamtgewicht der Zusammensetzung verwendet wird.

4. Verwendung nach einem der vorhergehenden Ansprüche, **dadurch gekennzeichnet, dass** der Inhibitor mindestens eines Caiciumkanals in einer Menge von 0,001 bis 5 % des Gesamtgewicht der Zusammensetzung verwendet wird.

5. Verwendung nach einem der vorhergehenden Ansprüche, **dadurch gekennzeichnet, dass** der Inhibitor mindestens eines Calciumkanals Verapamil ist.

6. Nichttherapeutisches Verfahren zur kosmetischen Behandlung von Falten und/oder Fältchen, das darin besteht, auf topischem Wege eine kosmetische Zusammensetzung aufzutragen, die in einem physiologisch akzeptablen Medium eine wirksame Menge mindestens eines Inhibitors mindestens eines Calciumkanals enthält, ausgewählt aus Verapamil, Arupamil, Gallopamil, Devapamil, Falipamil, Tiapamil, Nifedipin, Amiodipin, Dazodipin, Felodipin, Isradipin, Nicardipin, Nimodipin, Nisoldipin, Nitrendipin, Ryosidin, Diltiazem und Cinnarizin.
